# EUROPEAN PATENT SPECIFICATION

(11) **EP 0 533 228 B1**
(45) Date of publication and mention of the grant of the patent: **06.11.1996**
(21) Application number: 92202517.6
(22) Date of filing: 17.08.1992
(51) Int. Cl.: C07C 1/04, C10G 2/00, B01J 37/18

(54) **Process for the activation of a catalyst**
Verfahren zum Aktivieren eines Katalysators
Procédé d'activation d'un catalyseur

(30) Priority: 20.08.1991 GB 9117948
(43) Date of publication of application: 24.03.1993
(73) Proprietor: SHELL INTERNATIONALE RESEARCH MAATSCHAPPIJ B.V., 2596 HR Den Haag (NL)
(72) Inventor: Hu, Michael Chiun-Kuei, NL-1031 CM Amsterdam (NL); Ansorge, Joachim, NL-2596 HR The Hague (NL)

(56) References cited:
- EP-A- 0 152 652
- EP-A- 0 168 894
- WO-A-92/06784
- US-A- 2 644 829
- US-A- 4 492 774
- US-A- 4 670 414

## Description

The present invention relates to a process for the activation of a catalyst, in particular to a process for the activation of a catalyst of use in Fischer-Tropsch synthesis.

The preparation of hydrocarbons from a mixture of hydrogen and carbon monoxide at elevated temperature and pressure in the presence of a catalyst is referred to in the literature as the Fischer-Tropsch hydrocarbon synthesis.

Catalysts used in Fischer-Tropsch synthesis typically comprise one or more metals from Group VIII of the Periodic Table, optionally together with one or more promoters, and a support material or carrier. Particular interest exists in Fischer-Tropsch catalysts which comprise cobalt, especially in catalysts comprising cobalt in association with one or more promoters. Cobalt-containing catalysts have found particular application in the Fischer-Tropsch synthesis of hydrocarbons, yielding products consisting virtually completely of unbranched hydrocarbons with a high degree of selectivity to C₅+ hydrocarbons.

Before a catalyst can be used in Fischer-Tropsch synthesis, it must first be activated. Activation is effected by contacting the catalyst with a hydrogen-containing gas. The action of the activation step is to reduce the oxides of the catalytically active metal and oxides of other metals present as promoters in the catalyst. Such activation procedures, applicable to the activation of fresh catalyst and also in the procedures for regenerating or reactivating exhausted catalyst, are known in the art.

Thus, US Patent No. 2,289,731 (US 2,289,731) discloses a process for the reactivation (regeneration) of exhausted Fischer-Tropsch catalysts in which the catalyst is contacted with hydrogen to remove paraffinic hydrocarbons and other deposits from the catalyst particles. US 2,289,731 further discloses that it is of advantage to expose the catalyst particles to the oxidizing action of an oxygen-containing gas prior to being treated with hydrogen.

European Patent Application publication No. 0 168 894 (EP-A-0 168 894) discloses a process for the activation of a cobalt/zirconium/silica catalyst in which the catalyst particles are contacted with a hydrogen-containing gas at a temperature of between 200 and 350 °C and a hydrogen partial pressure between 0.001 and 75 bar, the hydrogen partial pressure being increased gradually or stepwise from an initial value to a final value such that the final value is at least five times the initial value.

Finally, US Patent No. 4,670,414 (US 4,670,414) discloses a process for the conversion of synthesis gas into hydrocarbons with a catalyst prepared by subjecting a cobalt carbonyl-impregnated alumina or silica support to an activation procedure comprising the steps, in sequence, of (A) reduction in hydrogen, (B) oxidation in an oxygen-containing gas, and (C) reduction in hydrogen, the activation procedure being conducted at a temperature below 500 °C. The catalyst is preferably slowly reduced in the presence of hydrogen. The reduction step can be conducted initially using a gaseous mixture comprising 5% hydrogen and 95% nitrogen, and thereafter, the concentration of hydrogen can be gradually increased until pure hydrogen is obtained. The reduced catalyst is then passivated at ambient temperature by contact with diluted air, after which the catalyst is slowly heated in diluted air to a temperature of from about 300 °C to about 350 °C. The thus oxidized catalyst is then reduced in the aforementioned manner.

In US 4,670,414 it is stated that the flow of reducing gas during the reduction stages of the procedure must be high enough so that any water formed has a partial pressure in the offgas of below 1%, in order to avoid excessive steaming of the exit end of the catalyst bed. Thus, to keep the water partial pressure to the required low level requires either the provision of equipment capable of handling a high throughput of gas, or operation of the reduction stages at low pressures, in turn requiring longer reduction periods.

A process has now been developed for the activation of a Fischer-Tropsch catalyst in which the catalyst is contacted with a hydrogen-containing gas, which process operates under a pressure regime which contacts the catalyst with the hydrogen-containing gas at high pressures. Most surprisingly, it has been found that this process yields an activated catalyst having a markedly increased activity, improved stability and a significantly higher selectivity to C₅+ hydrocarbons than corresponding catalysts activated by the prior art processes.

Accordingly, the present invention provides a process for the activation of a Fischer-Tropsch catalyst, which process comprises contacting the catalyst with a hydrogen-containing gas in a first stage at a pressure of up to 5 bar, rapidly increasing the pressure to at least 10 bar and contacting the catalyst with hydrogen-containing gas in a second stage at this pressure.

The hydrogen-containing gas used in the process of the present invention is substantially pure hydrogen gas or a mixture of hydrogen with one or more inert gases, for example nitrogen.

The action of the hydrogen-containing gas on the catalyst during the activation procedure is to reduce oxides of the catalytically active metal, and other metals present. This reduction yields water. As described in US 4,670,414, it is important that the partial pressure of water in the gas contacting the catalyst is kept at a low level, in order to avoid damaging the catalyst. Thus, the water partial pressure in the gas leaving the catalyst bed is preferably maintained at a level below 200 mbar, more preferably below 100 mbar. The maximum water partial pressure possible without damaging the catalyst will vary according to the specific catalyst selected, it having been found that some catalysts are more tolerant to the presence of water than others. The tolerance of the catalyst to the presence of water can readily be determined for a given catalyst by determining the activity of the catalyst in Fischer-Tropsch synthesis after contact with varying amounts of water in the activation procedure.

In order to keep the water partial pressure in the gas contacting the catalyst to a minimum, it is preferable to contact the catalyst initially with a hydrogen-containing gas containing a high level of inert gas and, thereafter, increase the hydrogen content of the gas stepwise or continuously whilst monitoring the water content of the gas leaving the catalyst bed. Typically, the catalyst will be initially contacted with a gas containing 0.5% v/v hydrogen, the hydrogen content being increased until substantially pure hydrogen is contacting the catalyst.

The first stage of the activation is conducted at a pressure of up to 5 bar, more preferably at a pressure of up to 3 bar. An operating pressure of about 3 bar is particularly preferred.

After contacting the catalyst with the hydrogen-containing gas in the first stage, the operating pressure is increased rapidly to a pressure of at least 10 bar, more preferably a pressure of at least 20 bar, even more preferably a pressure of at least 25 bar. An increase in pressure to about 25 bar is especially preferred. Thereafter, in the second stage of the process, the catalyst is contacted with the hydrogen-containing gas at this increased pressure.

It is preferred that the operating pressure of the process is maintained substantially constant during each of the two stages.

As a rapid increase in operating pressure, at a given concentration of water in the gas leaving the catalyst bed, will be accompanied by a corresponding rapid increase in the water partial pressure, the end of the first stage and the timing for the rapid increase in pressure will depend upon the degree of reduction of the catalyst achieved (as indicated by the water partial pressure in the gas leaving the catalyst bed) and the tolerance of the catalyst to the presence of water.

Thus, the first stage is ended and the operating pressure increased when the water content in the gas leaving the catalyst bed is such that the water partial pressure at the increased operating pressure is at a sufficiently low value, preferably below 200 mbar, more preferably below 100 mbar. Preferably, the hydrogen content of the hydrogen-containing gas is increased to substantially pure hydrogen during the first stage. The pressure of the substantially pure hydrogen is then rapidly increased. In this way, the catalyst is contacted with substantially pure hydrogen throughout the second stage.

The process of the present invention is carried out at an elevated temperature, preferably below 500 °C. More preferably, the process is operated at a temperature of from 100 °C to 350 °C, especially in the range of from 200 to 300 °C. A preferred temperature for operation of the process is about 250 °C.

The flowrate of the hydrogen-containing gas will depend upon the precise operating pressure being used and the tolerance of the catalyst to the presence of water; higher flowrates being required for the less water-tolerant catalysts under given process conditions. Typically the hydrogen-containing gas is provided at a gas hourly space velocity (GHSV) of from 100 to 10000 Nl/l/h, preferably from 200 to 6000 Nl/l/h. A typical GHSV for the hydrogen-containing gas is about 6000 Nl/l/h. However, GHSV values of from 300 to 1000 Nl/l/h may also be used.

The length of time that the catalyst is subjected to the stages of the process will again depend upon the precise operating conditions and the degree of reduction required, as indicated by the water content of the gas leaving the catalyst bed; a low water content indicating that a high degree of reduction has been achieved. Typically, the first and second stages are each operated for a period of from 5 to 25 hours, giving a typical total process duration of 10 to 50 hours.

Between the first and second stages the operating pressure is increased rapidly from the relatively low operating pressure of the first stage to the high pressure of the second stage. In this specification, the term "rapidly" is to be taken as a reference to a period of time that is short in comparison to the overall duration of the process. The pressure is preferably increased over a period of time that is as short as possible. It will of course be appreciated that the plant equipment in which the process is operated will often place constraints on the maximum rate at which the pressure can be increased. If possible, the pressure is increased over a period ranging in length from 1 second to 30 minutes, typical periods ranging in length from 5 seconds to 20 minutes.

The process of the present invention may be applied to activate a fresh catalyst, prior to its use in a Fischer-Tropsch synthesis. Alternatively, the process may be applied in the regeneration (reactivation) of an exhausted or partially exhausted catalyst.

In this respect, the term "activation" as used herein is to be taken as a reference to the activation of a fresh catalyst, prior to its use and to the regeneration (reactivation) of an exhausted or partially exhausted catalyst, unless otherwise stated. Further, the process of the present invention is most advantageously applied in the reduction/oxidation/reduction, or the so-called "ROR-activation" procedure described in US 4,670,414. The ROR-activation procedure may be advantageously applied in the activation of a fresh catalyst or the regeneration (reactivation) of an exhausted or partially exhausted catalyst.

Thus, according to a further aspect of the present invention, there is provided a process for the activation of a Fischer-Tropsch catalyst comprising the steps of a) contacting the catalyst with a hydrogen-containing gas; b) contacting the catalyst with a gas having oxidizing activity; and c) contacting the catalyst with a hydrogen-containing gas; characterized in that a process as hereinbefore described is employed in at least one of steps a) and c).

Either one or both of the reduction stages in the ROR-activation procedure may comprise the process of the present invention. If the process is employed during only one of the reduction stages of the ROR-activation procedure, it is most preferably used during the second reduction stage.

In the first stage in the ROR-activation procedure, the catalyst particles are contacted with a hydrogen-containing gas. The process as hereinbefore described may be employed as the first stage in the ROR-activation procedure. Alternatively, a known reduction procedure may be used, such as described in US 4,670,414. The hydrogen-containing gas used in such a procedure may be substantially pure hydrogen or may comprise hydrogen diluted by one or more inert gases, such as nitrogen. The hydrogen-containing gas may be supplied at a pressure of from 1 to 30 bar, for example about 25 bar. The catalyst is preferably contacted with the gas at a temperature below 500 °C, preferably at a temperature of from 100 to 400 °C, typically from 150 to 350 °C, and at a GHSV of from 100 to 10000 Nl/l/h, more preferably from 200 to 6000 Nl/l/h, for a period of from 1 to 50 hours.

The second stage of the ROR-activation procedure is an oxidation stage, in which the catalyst is contacted with a gas having an oxidizing action. The gas used in this stage is conveniently oxygen or an oxygen-containing gas, for example air. The reactions occurring during the oxidation stage are exothermic. In order to avoid an excessive rise in temperature which could damage the catalyst, it is preferred to contact the catalyst with air, further diluted with nitrogen. Typically the gas contains from about 1 to 5% v/v, preferably about 3% v/v oxygen. The temperature at which the oxidation is effected may be in the range of from 100 to 400 °C, preferably from 150 to 350 °C. The catalyst is contacted with the oxygen-containing gas at a pressure of from 1 to 25 bar, typically about 10 bar, at a GHSV of from about 100 to 5000 Nl/l/h, typically from 500 to 1000 Nl/l/h, for a period of from 1 to 30 hours.

The third stage of the ROR-activation procedure is a final reduction of the oxidized catalyst produced in the second stage described above. For this third stage, if the process of the present invention, as hereinbefore described, has been employed in the first stage of the ROR-activation, the known reduction process described above in relation to the first stage may be employed. Most preferably, the process of the present invention is employed in the third stage of the ROR-activation procedure.

In a further aspect, the present invention provides a Fischer-Tropsch catalyst whenever activated by a process as hereinbefore described.

The process of the present invention may be applied as a one-pass process, that is, a process in which the hydrogen-containing gas fed to the catalyst bed contacts the catalyst only once. In a preferred embodiment, the gas leaving the catalyst bed is dried to reduce the water content of the gas, recompressed to the process operating pressure and recycled to the inlet for the catalyst bed. In a particularly preferred embodiment, the dried, hydrogen-containing gas being recycled, together with the fresh, hydrogen-containing feed gas, is heated by recovering heat from the gas leaving the catalyst bed.

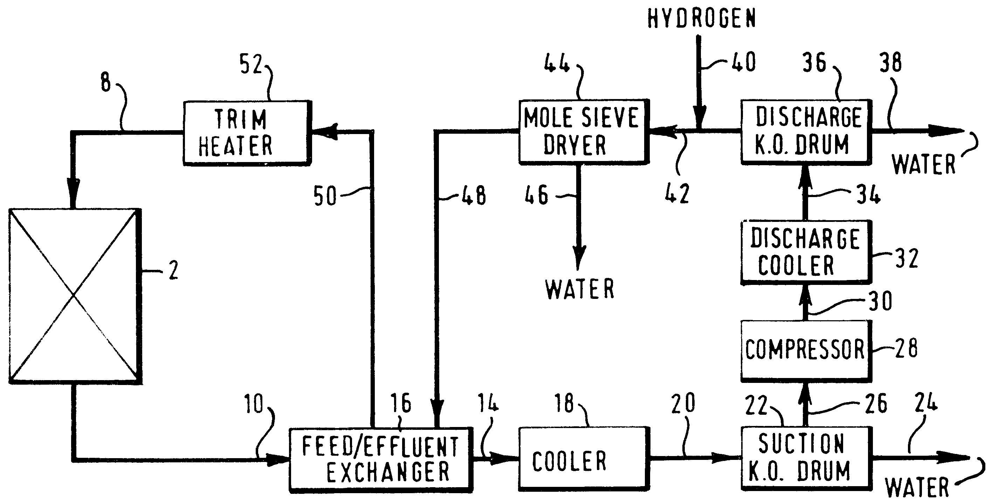

The process of the present invention will be further described with reference to the accompanying figure which is a schematical diagram of a preferred arrangement of apparatus for conducting the process of the present invention.

Referring to the Figure, a reactor vessel 2 has an inlet line 8 and an outlet line 10. Advantageously, fresh catalyst is loaded into a catalyst bed within the reactor 2 prior to being activated by the process of the present invention. The catalyst may also be regenerated (reactivated) whilst remaining in the reactor vessel 2. The catalyst may be regenerated (reactivated) several times whilst remaining in the reactor vessel 2, throughout its useful life.

During the process of the present invention, hydrogen-containing feed gas enters the reactor vessel 2 from the inlet line 8 and contacts the catalyst bed. Effluent gas exiting the catalyst bed leaves the reactor vessel 2 and enters the outlet line 10. The effluent gas in the outlet line 10 is depleted in hydrogen and is rich in water-vapour, relative to the feed gas in the inlet line 8. From the outlet line 10, the effluent gas flows to a feed/effluent heat exchanger 16 to undergo a first stage of cooling and then, via line 14, to a further cooler 18 to undergo a second stage of cooling in preparation for compression. The effluent gas leaves the cooler 18 and, through line 20, enters a suction knock-out drum 22. In the suction knock-out drum 22, water, now present as droplets entrained in the effluent gas, is removed from the effluent gas stream and leaves the suction knock-out drum 22 through line 24. From the suction knock-out drum 22, the effluent gas is fed, via line 26, to the inlet of a compressor 28. The compressed effluent gas leaves the compressor 28 via line 30, is cooled in a discharge cooler 32 and fed, via line 34, to a discharge knock-out drum 36. The action of compression and cooling on the effluent gas stream causes further droplets of water to form, which collect and are removed from the discharge knock-out drum 36 through line 38. From the discharge knock-out drum 36, the effluent gas is mixed with fresh hydrogen-containing gas from line 40 and the combined gas stream fed, through line 42, to a molecular sieve dryer 44.

The molecular sieve dryer 44 may typically contain an aluminosilicate adsorbant for removing water from the combined gas stream. Water is shown, schematically, leaving the molecular sieve dryer 44 through line 46 and the dried gas stream is shown leaving through line 48.

The dried gas stream is heated by heat exchange with the effluent gas leaving the reactor 2 in the feed/effluent exchanger 16. From the feed/effluent exchanger 16, the gas stream is fed, via line 50, to a trim heater 52 to bring the gas up to the process operating temperature. The hot gas enters the reactor 2 from the trim heater 52 through line 8.

The apparatus represented in the Figure may be used to service a plurality of reactor vessels (not shown), each of which is isolated by valves and which may, in turn, be connected to the process apparatus shown in the Figure for activation or regeneration (reactivation) of the catalyst therein.

A bypass line (not shown) may be provided between the two lines 8, 10 to allow gas to bypass the reactor vessel 2 and be removed from the process apparatus as a purge.

The process of the present invention may be applied to any Fischer-Tropsch catalyst. Fischer-Tropsch catalysts frequently comprise, as the catalytically active component, a metal from Group VIII of the Periodic Table of Elements. Particular catalytically active metals include iron, cobalt and nickel. The process of the present invention is particularly advantageous when applied to Fischer-Tropsch catalysts comprising cobalt as the catalytically active metal.

The catalytically active metal is preferably supported on a porous carrier. The porous carrier may be selected from any suitable refractory metal oxide or silicates or a combination thereof. Particular examples of preferred carriers include silica alumina, titania or mixtures thereof. Most preferably, a porous silica carrier is used. The active metal may be applied to the carrier by any of the techniques well known in the art, for example kneading, impregnation or precipitation. Impregnation is a particularly preferred technique, which may be carried out by contacting the carrier with a compound of the active metal in the presence of a liquid, most conveniently in the form of a solution of the metal compound. The compound of the active metal may be inorganic or organic. Inorganic compounds of the active metal are preferred, in particular nitrates. The liquid used may also be either organic or inorganic, with water being a particularly preferred and convenient liquid.

The amount of catalytically active metal on the carrier is preferably from 3 to 100 pbw per 100 pbw of carrier material, more preferably from 10 to 80 pbw, especially from 20 to 60 pbw.

If desired, the catalyst may also comprise one or more metals or metal oxides as promoters. Suitable metal oxide promoters may be selected from groups IIa, IIIb, IVb, Vb and VIb of the Periodic Table, or the actinides and lanthanides. In particular, oxides of magnesium, calcium, strontium, barium, scandium, yttrium, lanthanum, cerium, titanium, zirconium, hafnium, thesium, uranium, vanadium and chromium are most suitable. A particularly preferred metal oxide promoter is zirconium oxide. Suitable metal promoters may be selected from groups VIIb or VIII of the Periodic Table. Rhenium and group VIII noble metals are particularly suitable, with ruthenium, platinum and palladium being especially preferred. The promoter may be applied to the porous carrier either before or after application of the catalytically active metal. The amount of promoter present is preferably from 0.1 to 150 pbw per 100 pbw of carrier. A particularly preferred catalyst is a cobalt/zirconium/silica catalyst.

Examples of suitable catalysts to which the process of the present invention may be applied are disclosed in European Patent Applications publication Nos. EP 0 104 672, EP 0 110 449, EP 0 127 220, EP 0 167 215, EP 0 180 269 and EP 0 221 598. Examples of very suitable processes for preparing such catalysts are disclosed in UK patent applications Nos. GB 8918845.2 and GB 8925979.0, forming priority for published European Patent Application publication Nos. EP 0 421 502 and EP 0 428 223 respectively.

The Fischer-Tropsch catalyst, once activated by the process of the present invention is suitable for use in a process for the synthesis of hydrocarbons from a mixture of carbon monoxide and hydrogen, which mixture is commonly referred to as synthesis gas. The conversion of the mixture of hydrogen and carbon monoxide may be carried out at a temperature of from 125 to 350 °C, preferably from 175 to 250 °C, and at a pressure of from 5 to 100 bar, more preferably from 10 to 50 bar. In the process, the catalyst may be contacted with a synthesis gas having a hydrogen to carbon monoxide molar ratio less than 2.5, preferably less than 1.75. More preferably, the hydrogen to carbon monoxide molar ratio of the synthesis gas is in the range of from 0.4 to 1.5, especially from 0.9 to 1.3.

It has been found that the process of the present invention, when applied to fresh catalyst or to exhausted or partially exhausted catalyst, significantly increases the activity, selectivity and stability of the catalyst in Fischer-Tropsch synthesis. In particular, using as a basis for comparison the process operating temperature of the Fischer-Tropsch synthesis necessary to achieve a space time yield of 100 g/l/h (100 STY temperature), it has been found that the process of the present invention yields a catalyst having a lower 100 STY temperature, indicating a higher activity, than catalysts activated using procedures known from the prior art. Further, the selectivity of the cobalt-containing catalyst to C₅+ hydrocarbons is significantly improved by use of the process of the present invention.

The process of the present invention is further illustrated by the following examples.

### EXAMPLE 1 - Catalyst Preparation

A catalyst was prepared by the following method:
Precipitated silica (Sipernat 50 (Trade Mark) ex. Degussa, primary particle size 6-7 nm, average agglomerate size 50 µm, surface area 450 m2/g) (400 kg), ammonium zirconium carbonate (Bacote 20 (Trade Mark), 19% w/w ZrO₂) (280 kg) and water (680 kg) were mixed in a mulling machine operated at low speed.

The resulting mixture was mulled at high speed for 20 minutes. Aqueous acetic acid (28.6 kg of 70% w/w solution) and water (77 kg) were added and the resulting mixture further mulled at high speed for 10 minutes. The resulting mixture had a loss on ignition of 73%. Polyelectrolyte (Nalco (Trade Mark), 4% by weight of total mixture) was added and the mixture mulled at high speed for a further five minutes.

The resulting mixture was extruded through a Delrin dieplate to yield trilobe extrudates having a nominal diameter of 1.35 mm. The extrudates were dried at a temperature of 330 to 350 °C, and calcined for a period of 1 hour at a temperature of 800 °C.

To an aqueous solution of cobalt nitrate (516 kg of a 14% by weight solution) was added cobalt nitrate hexahydrate (1100 kg) and the solution stirred. The extrudates were immersed in this solution to effect impregnation of the extrudates with cobalt. The resulting extrudates were dried at a temperature of 350 °C and finally calcined at a temperature of from 250 to 500 °C.

### EXAMPLE 2

Catalyst prepared as described in Example 1 was loaded into a fixed bed reactor and activated as follows:

A mixture of hydrogen (1% v) in nitrogen was fed to the top of the catalyst bed at a temperature of 250 °C, a pressure of 3 bar and at a gas hourly space velocity (GHSV) of 6000 Nl/l/h. The water content of the exhaust gas leaving the bottom of the catalyst bed was monitored and the hydrogen content of the feed gas increased gradually to 100% v, whilst maintaining the water content of the exhaust gas below about 8500 ppmv (25.5 mbar).

Once the hydrogen content of the gas reached 100% v, the water content of the exhaust gas began to fall. At a water content in the exhaust gas of about 1000 ppmv, the gas pressure was increased sharply over a period of 15 minutes to 25 bar, yielding a water partial pressure of 150 mbar in the exhaust gas. These conditions were maintained until a total activation period of 24 hours had been achieved.

The activated catalyst was then contacted with a synthesis gas feed comprising hydrogen and carbon monoxide in a ratio of about 1:1 at an inlet pressure of 25 bar and at a GHSV of 800 Nl/l/h. A heavy wax was produced.

As a means of monitoring the performance of the catalyst, the temperature of the catalyst bed was continually monitored and adjusted to give a space time yield of 100 (100 STY temperature). Further, the selectivity of the catalyst to C₅ compounds and heavier (C₅+ selectivity) was also monitored. The 100 STY temperature and C₅+ selectivity of the catalyst over a reaction period of 135 hours are given in Table I.

**TABLE I**

| Time (hours) | 100 STY temperature (°C) | C₅+ selectivity (% wt) |
|---|---|---|
| 21 | 207.4 | 89.8 |
| 25 | 208.1 | 89.4 |
| 29 | 208 | 89.2 |
| 39 | 208.9 | 89.3 |
| 42 | 208.8 | 89.2 |
| 46 | 209.2 | 89.1 |
| 53 | 209.9 | 88.9 |
| 57 | 210.5 | 88.7 |
| 59 | 209.3 | 88.9 |
| 60 | 209.9 | 88.9 |
| 65 | 210.3 | 88.9 |
| 77 | 212 | 88.3 |
| 85 | 211.9 | 88.4 |
| 92 | 212.4 | 88.4 |
| 103 | 212.2 | 88.2 |
| 117 | 213.1 | 88.6 |
| 135 | 213.2 | 88.2 |

### EXAMPLE 3

At the end of the reaction period, the catalyst of Example 2 was subjected to a reactivation procedure as follows:

To remove the accumulated wax in the catalyst, the catalyst bed was contacted with hydrogen to effect a hydrogen stripping. A mixture of hydrogen (5% v) in nitrogen at a GHSV of 700 Nl/l/h, a temperature of 250 °C and a pressure of 10 bar was fed to the top of the catalyst bed for a period of 24 hours.

Oxidation of the catalyst was then effected by feeding a mixture of oxygen (0.5% v as 2.5% v air) in nitrogen at a GHSV of 700 Nl/l/h, at a temperature of 250 °C and a pressure of 10 bar to the top of the catalyst bed for a period of 24 hours.

The oxidized catalyst was then activated following the general procedure set out in Example 2.

The activated catalyst was then contacted again with a synthesis gas feed under the general reaction conditions set out in Example 2. The 100 STY temperature and C₅+ selectivity of the catalyst over a reaction period of 165 hours were determined, the results of which are set out in Table II.

**TABLE II**

| Time (hours) | 100 STY temperature (°C) | C₅+ selectivity (% wt) |
|---|---|---|
| 20 | 202.6 | 91.4 |
| 33 | 204.2 | 90.9 |
| 36 | 203.9 | 90.9 |
| 38 | 205.3 | 90.9 |
| 48 | 206.1 | 90.5 |
| 51 | 205.8 | 90.3 |
| 53 | 206.0 | 90.5 |
| 61 | 206.6 | 90.2 |
| 67 | 207.5 | 90.2 |
| 74 | 207.5 | 90.2 |
| 90 | 208.8 | 90.1 |
| 106 | 209.4 | 89.9 |
| 138 | 211.0 | 89.5 |
| 160 | 211.5 | 89.5 |
| 165 | 210.6 | 89.9 |

### EXAMPLE 4

After the reaction period, the catalyst of Example 3 was treated again to the general reactivation procedure set out in Example 3. The catalyst was then once again contacted with a synthesis gas feed under the general reaction conditions set out in Example 2 for a reaction period of 181 hours. The 100 STY temperature and C₅+ selectivity were continually monitored, the results of which are set out in Table III.

**TABLE III**

| Time (hours) | 100 STY temperature (°C) | C₅+ selectivity (% wt) |
|---|---|---|
| 29 | 202.5 | 91.6 |
| 31 | 202.9 | 91.4 |
| 58 | 204.7 | 90.7 |
| 62 | 204.7 | 90.9 |
| 81 | 205.5 | 90.7 |
| 83 | 206.0 | 90.7 |
| 112 | 207.0 | 90.6 |
| 116 | 207.1 | 90.8 |
| 124 | 207.7 | 90.8 |
| 130 | 207.8 | 90.6 |
| 148 | 207.7 | 90.8 |
| 152 | 208.1 | 90.8 |
| 160 | 207.7 | 90.6 |
| 168 | 207.5 | 90.7 |
| 171 | 208.5 | 90.8 |
| 175 | 207.2 | 91.0 |
| 181 | 207.5 | 90.9 |

### EXAMPLE 5 - Comparative Example

For comparative purposes a sample of the catalyst was activated following the general procedure of Example 2, with the exception that once the hydrogen content of the gas had reached 100% v the pressure was maintained at 3 bar for the remainder of the activation period of 24 hours. The activated catalyst was then contacted with synthesis gas under the general reaction conditions set out in Example 2. The 100 STY temperature and C₅+ selectivity of the catalyst during the reaction period are set out in Table IV.

The catalyst was then reactivated following the general procedure of Example 3, with the exception that the final activation stage followed the procedure set out above, that is, once the hydrogen content of the gas had reached 100% v the pressure was maintained at 3 bar for the remainder of the activation period.

The 100 STY temperature and C₅+ selectivity of the reactivated catalyst under the general reaction conditions of Example 2 were obtained, the results of which are set out in Table V.

The catalyst was then subjected to a further similar reactivation procedure with the final activation stage modified as before and a further reaction period. The 100 STY temperature and C₅+ selectivity of the catalyst are set out in Table VI.

**TABLE IV**

| Time (hours) | 100 STY temperature (°C) | C₅+ selectivity (% wt) |
|---|---|---|
| 4 | 201.0 | 92.0 |
| 10 | 203.0 | 91.0 |
| 15 | 211.0 | 89.6 |
| 41 | 212.0 | 88.2 |
| 85 | 214.0 | 88.0 |
| 92 | 220.0 | 87.2 |

**TABLE V**

| Time (hours) | 100 STY temperature (°C) | C₅+ selectivity (% wt) |
|---|---|---|
| 25 | 208.8 | 90.2 |
| 28 | 209.2 | 89.9 |
| 69 | 210.9 | 89.5 |

**TABLE VI**

| Time (hours) | 100 STY temperature (°C) | C₅+ selectivity (% wt) |
|---|---|---|
| 46 | 210 | 89.9 |
| 78 | 214 | 89.2 |
| 102 | 215 | 89.2 |
| 130 | 214 | 88.2 |
| 142 | 214 | 88.4 |
| 227 | 215 | 89.0 |
| 282 | 216 | 89.2 |
| 321 | 217 | 89.2 |
| 329 | 218 | 88.8 |
| 351 | 220 | 88.5 |
| 379 | 221 | 88.1 |
| 385 | 221 | 88.2 |
| 399 | 220 | 87.8 |
| 402 | 220 | 88.3 |
| 405 | 219 | 87.9 |
| 422 | 220 | 88.0 |

A comparison of the data set out in Tables I to III with the corresponding data of Tables IV to VI indicates the improvements in catalyst performance achieved by using the activation process of the present invention. In particular, it can be seen that the process of the present invention yields an active catalyst having a superior level of activity (indicated by a lower 100 STY temperature) than a catalyst prepared by a conventional activation process. Further, the process of the present invention gives rise to an active catalyst having an improved stability and a significantly increased selectivity to C₅+ products in Fischer-Tropsch synthesis.

### EXAMPLE 6

A catalyst was prepared using the general procedure set out in Example 1, with the additional steps of immersing the catalyst particles in an aqueous solution of zirconium nitrate, drying and calcining at a temperature of from 250 to 500 °C.

The catalyst thus obtained was activated using the general procedure of the prior art outlined in Example 5 and contacted with synthesis gas under the general reaction conditions set out in Example 2. The 100 STY temperature and C₅+ selectivity of the catalyst during the reaction period are set out in Table VII.

The catalyst was then reactivated following the general procedure of the prior art outlined in Example 5 and the 100 STY temperature and C₅+ selectivity of the reactivated catalyst under the general reaction conditions of Example 2 were obtained, the results of which are set out in Table VIII.

Finally, the catalyst was subjected to a further reactivation following the general procedure set out in Example 3, that is a reactivation process according to the present invention. The reactivated catalyst was again contacted with synthesis gas under the general reaction conditions of Example 2 and the 100 STY temperature and C₅+ selectivity obtained. The results are set out in Table IX.

**TABLE VII**

| Time (hours) | 100 STY temperature (°C) | C₅+ selectivity (% wt) |
|---|---|---|
| 18 | 208.5 | 89.1 |
| 31 | 208.7 | 87.3 |
| 42 | 210.5 | 86.9 |

**TABLE VIII**

| Time (hours) | 100 STY temperature (°C) | C₅+ selectivity (% wt) |
|---|---|---|
| 48 | 206.1 | 89.3 |
| 51 | 205.8 | 88.8 |
| 66 | 207.5 | 88.0 |
| 90 | 208.8 | 88.4 |

**TABLE IX**

| Time (hours) | 100 STY temperature (°C) | C₅+ selectivity (% wt) |
|---|---|---|
| 84 | 206.0 | 90.1 |
| 100 | 207.2 | 90.3 |
| 130 | 207.8 | 90.0 |
| 181 | 207.5 | 89.9 |

From a comparison of the results set out in Tables VII to IX, it can be seen that the application to the catalyst of an activation procedure according to the present invention leads to a significant increase in both activity (lower 100 STY temperature), C₅+ selectivity and stability of the catalyst compared with a catalyst activated using the prior art procedures.

## Claims

1. A process for the activation of a Fischer-Tropsch catalyst, which process comprises contacting the catalyst with a hydrogen-containing gas in a first stage at a pressure of up to 5 bar, rapidly increasing the pressure to at least 10 bar and contacting the catalyst with a hydrogen-containing gas in a second stage at this pressure.

2. A process according to claim 1, characterized in that the first stage is operated at a pressure of up to 3 bar.

3. A process according to either of claims 1 or 2, characterized in that the second stage is operated at a pressure of at least 20 bar, preferably at least 25 bar.

4. A process according to any preceding claim, characterized in that during the first stage the hydrogen content of the hydrogen-containing gas is increased from 1% v/v to 100% v/v.

5. A process according to any preceding claim, characterized in that during the second stage the catalyst is contacted with substantially pure hydrogen.

6. A process according to any preceding claim, characterized in that the water partial pressure in the gas leaving the catalyst is maintained below 200 mbar, preferably below 100 mbar.

7. A process according to any preceding claim, characterized in that the process operating temperature is in the range of from 100 to 350 °C.

8. A process according to any preceding claim, characterized in that the gas hourly space velocity of the gas is in the range of from 100 to 10000 Nl/l/h.

9. A process according to any preceding claim, characterized in that the pressure is increased over a period of time of from 5 seconds to 20 minutes.

10. A process according to any preceding claim, characterized in that gas leaving the catalyst is dried, combined with fresh hydrogen-containing gas and recycled to further contact the catalyst.

11. A process for the activation of a Fischer-Tropsch catalyst comprising the steps of:
a) contacting the catalyst with a hydrogen-containing gas;
b) contacting the catalyst with a gas having oxidizing activity; and
c) contacting the catalyst with a hydrogen-containing gas;
characterized in that a process according to any one of claims 1 to 10 is employed in at least one of steps a) and c), preferably in at least step c).

12. A process according to any preceding claim, characterized in that the catalyst comprises silica, alumina, titania or mixtures thereof as carrier.

13. A process according to any preceding claim, characterized in that the catalyst comprises cobalt as a catalytically active metal.

14. A process according to any preceding claim, characterized in that the catalyst comprises, as promoter, zirconium.

15. A Fischer-Tropsch catalyst activated by a process according to any preceding claim.

16. A process for the preparation of hydrocarbons comprising contacting a mixture of carbon monoxide and hydrogen with a catalyst according to claim 15.

## Patentansprüche

1. Verfahren zur Aktivierung eines Fischer-Tropsch-Katalysators, bei dem man in einer ersten Stufe den Katalysator bei einem Druck von bis zu 5 bar mit einem wasserstoffhaltigen Gas in Berührung bringt, den Druck schnell auf mindestens 10 bar erhöht und in einer zweiten Stufe den Katalysator bei diesem Druck mit einem wasserstoffhaltigen Gas in Berührung bringt.

2. Verfahren nach Anspruch 1, dadurch gekennzeichnet, daß man die erste Stufe bei einem Druck von bis zu 3 bar durchführt.

3. Verfahren nach Anspruch 1 oder 2, dadurch gekennzeichnet, daß man die zweite Stufe bei einem Druck von mindestens 20 bar, vorzugsweise mindestens 25 bar, durchführt.

4. Verfahren nach einem der vorhergehenden Ansprüche, dadurch gekennzeichnet, daß man während der ersten Stufe den Wasserstoffgehalt des wasserstoffhaltigen Gases von 1 Vol.-% auf 100 Vol.-% erhöht.

5. Verfahren nach einem der vorhergehenden Ansprüche, dadurch gekennzeichnet, daß man während der zweiten Stufe den Katalysator mit weitgehend reinem Wasserstoff in Berührung bringt.

6. Verfahren nach einem der vorhergehenden Ansprüche, dadurch gekennzeichnet, daß man den Wasserpartialdruck des den Katalysator verlassenden Gases unter 200 mbar, vorzugsweise unter 100 mbar, hält.

7. Verfahren nach einem der vorhergehenden Ansprüche, dadurch gekennzeichnet, daß man es bei einer Temperatur im Bereich von 100 bis 350°C durchführt.

8. Verfahren nach einem der vorhergehenden Ansprüche, dadurch gekennzeichnet, daß man eine Katalysatorbelastung im Bereich von 100 bis 10000 Nl/l/h wählt.

9. Verfahren nach einem der vorhergehenden Ansprüche, dadurch gekennzeichnet, daß man den Druck innerhalb von 5 Sekunden bis 20 Minuten erhöht.

10. Verfahren nach einem der vorhergehenden Ansprüche, dadurch gekennzeichnet, daß man das den Katalysator verlassende Gas trocknet, mit frischem wasserstoffhaltigem Gas vermischt und zum weiteren Kontakt mit dem Katalysator zurückführt.

11. Verfahren zur Aktivierung eines Fischer-Tropsch-Katalysators, das folgende Schritte umfaßt:
a) Inberührungbringen des Katalysators mit einem wasserstoffhaltigen Gas;
b) Inberührungbringen des Katalysators mit einem oxidationsaktiven Gas, und
c) Inberührungbringen des Katalysators mit einem wasserstoffhaltigen Gas,
dadurch gekennzeichnet, daß man in mindestens einem der Schritte a) und c), vorzugsweise mindestens in Schritt c), ein Verfahren nach einem der Ansprüche 1-10 anwendet.

12. Verfahren nach einem der vorhergehenden Ansprüche, dadurch gekennzeichnet, daß der Katalysator Siliziumoxid, Aluminiumoxid, Titanoxid oder deren Gemische als Träger enthält.

13. Verfahren nach einem der vorhergehenden Ansprüche, dadurch gekennzeichnet, daß der Katalysator Kobalt als katalytisch aktives Metall enthält.

14. Verfahren nach einem der vorhergehenden Ansprüche, dadurch gekennzeichnet, daß der Katalysator Zirkon als Promotor enthält.

15. Fischer-Tropsch-Katalysator, aktiviert nach einem Verfahren nach einem der vorhergehenden Ansprüche.

16. Verfahren zur Herstellung von Kohlenwasserstoffen, bei dem man ein Gemisch aus Kohlenmonoxid und Wasserstoff mit einem Katalysator nach Anspruch 15 in Berührung bringt.

## Revendications

1. Procédé pour l'activation d'un catalyseur de Fischer-Tropsch, ce procédé comprenant la mise en contact du catalyseur avec un gaz contenant de l'hydrogène, dans un premier stade, sous une pression allant jusqu'à 5 bars, l'élévation rapide de la pression jusqu'à au moins 10 bars, et la mise en contact du catalyseur avec un gaz contenant de l'hydrogène dans un deuxième stade, sous cette pression.

2. Procédé selon la revendication 1, caractérisé en ce que l'on fait fonctionner le premier stade sous une pression allant jusqu'à 3 bars.

3. Procédé selon la revendication 1 ou 2, caractérisé en ce que l'on fait fonctionner le deuxième stade sous une pression d'au moins 20 bars, de préférence d'au moins 25 bars.

4. Procédé selon l'une quelconque des revendications précédentes, caractérisé en ce que, durant le premier stade, la teneur en hydrogène du gaz contenant de l'hydrogène passe de 1% en volume à 100% en volume.

5. Procédé selon l'une quelconque des revendications précédentes, caractérisé en ce que, durant le deuxième stade, le catalyseur est mis en contact avec de l'hydrogène essentiellement pur.

6. Procédé selon l'une quelconque des revendications précédentes, caractérisé en ce que la pression partielle de l'eau dans le gaz quittant le catalyseur est maintenue au-dessous de 200 mbars, de préférence au-dessous de 100 mbars.

7. Procédé selon l'une quelconque des revendications précédentes, caractérisé en ce que la température de fonctionnement du procédé est dans la gage de 100 à 350°C.

8. Procédé selon l'une quelconque des revendications précédentes, caractérisé en ce que la vitesse spatiale horaire du gaz est dans la gamme de 100 à 10 000 Nl/l/h (mesurée dans les conditions normales de température et de pression).

9. Procédé selon l'une quelconque des revendications précédentes, caractérisé en ce que la pression est augmentée sur une période de 5 secondes à 20 minutes.

10. Procédé selon l'une quelconque des revendications précédentes, caractérisé en ce que le gaz quittant le catalyseur est séché, combiné avec du gaz frais contenant de l'hydrogène et recyclé pour être par la suite remis en contact avec le catalyseur.

11. Procédé pour l'activation d'un catalyseur de Fischer-Tropsch, comprenant les étapes consistant à:
a) mettre le catalyseur en contact avec un gaz contenant de l'hydrogène;
b) mettre le catalyseur en contact avec un gaz ayant une activité oxydante; et
c) mettre le catalyseur en contact avec un gaz contenant de l'hydrogène;
caractérisé en ce que l'on emploie un procédé selon l'une quelconque des revendications 1 à 10 dans l'une au moins des étapes a) et c), de préférence au moins dans l'étape c).

12. Procédé selon l'une quelconque des revendications précédentes, caractérisé en ce que le catalyseur comprend, comme support, de la silice, de l'alumine, de l'oxyde de titane, ou leurs mélanges.

13. Procédé selon l'une quelconque des revendications précédentes, caractérisé en ce que le catalyseur comprend du cobalt comme métal à activité catalytique.

14. Procédé selon l'une quelconque des revendications précédentes, caractérisé en ce que le catalyseur comprend, comme promoteur, du zirconium.

15. Catalyseur de Fischer-Tropsch activé par un procédé selon l'une quelconque des revendications précédentes.

16. Procédé de préparation d'hydrocarbures, comprenant la mise en contact d'un mélange de monoxyde de carbone et d'hydrogène avec un catalyseur selon la revendication 15.
